# NEW EUROPEAN PATENT SPECIFICATION

(11) **EP 0 879 048 B2**
(45) Date of publication and mention of the opposition decision: **29.07.2009**
(45) Mention of the grant of the patent: 28.04.2004
(21) Application number: 97904514.3
(22) Date of filing: 10.02.1997
(51) Int. Cl.: A61K 9/20, A61K 47/26

(54) **Method for making tablets using trehalose**
Verfahren zur Herstellung von Tabletten mit Hilfe von Trehalose
Procédé de préparation de tablettes en utilisant de la trehalose

(30) Priority: 09.02.1996 US 599277; 09.02.1996 US 599273
(43) Date of publication of application: 25.11.1998
(73) Proprietor: Quadrant Drug Delivery Limited, Nottingham, NG11 6JS (GB)
(72) Inventor: ROSER, Bruce, Joseph, Cambridge CB3 0LW (GB); BLAIR, Julian, St. Ives PE17 6JH (GB); COLACO, Camilo, Cambridge CB2 2JN (GB); HATLEY, Ross, Henry, Morris, Cambridge CB4 5LH (GB)
(74) Representative: Perry, Robert Edward
(86) International application number: PCT/GB1997/000367
(87) International publication number: WO 1997/028788

(56) References cited:
- EP- - 0 600 730
- EP-A- 0 691 407
- EP-A- 0 693 558
- WO- -96//4007
- WO- -95//24183
- US-A- 4 678 812
- US-A- 4 712 310
- US-A- 4 762 857
- DATABASE WPI Week 9436 Derwent Publications Ltd., London, GB; AN 94-289966 [36] XP002032298 & JP 06 217 716 A (AJINOMOTO) 9 August 1994
- ' Medical Dictionary', PHARMACEUTICAL MANUFACTURING REVIEW article ' Extract for "diluent"'

## Description

This invention relates to tablets.

Drugs and other active agents are most frequently administered orally by means of solid dosage forms. Large scale production methods used in their preparation usually require that these dosage forms contain other additives in addition to the active ingredients. These additives, generally known as excipients, may be included in the formulations to facilitate handling, to enhance the physical appearance, improve the stability and enhance the release or availability for absorption of the active agent or agents. The additives, ie excipients may include diluents, disintegrants, binders and lubricants.

Tablets are solid dosage forms which contain drugs or other active substances with or without suitable diluents.

Tablets are usually prepared by compression, extrusion, freeze drying or molding. Tablets provide advantages both to the manufacturer (e.g., simplicity and economy of preparation, stability and convenience in packaging, shipping and dispensing) and the patient (e.g., accuracy of dosage, compactness, portability, blandness of taste and ease of administration). Tablets are one of the most common form of solid dose drug delivery. For review see, Pogany et al. (1988) Acta Pharm. Hung. 58:49- 58:49-55; Doelker et al. (1988) Boll. Chim. Farm. 127:37-49; Hiestand et al. (1977) J. Pharm. Sci. 66:510-519; and Cooper et al. (1972) J. Pharm. Sci. 61:1511-1555.

Compressed tablets are usually prepared by large-scale production methods, while molded tablets generally involve smaller scale operations. Compressed tablets usually contain no special coating and are made from a small number of powdered, crystalline or excipients made by a granulation procedure alone or in combination with disintegrants, controlled-release polymers, binders, waxes, lubricants, diluents and, in many cases, colorants.

Compressed tablets may be coated with a variety of substances for a variety of reasons including alteration of their physical characteristics and modification of the rate and extent of release of active ingredients. A sugar coating may be applied. Such coatings are beneficial in masking drugs possessing objectionable tastes or odours and in protecting materials sensitive to humidity, light or oxidation. Tablets may also be covered with a thin layer or film of water soluble or insoluble material. Enteric coated tablets are coated with substances that resist dissolution in gastric fluid but disintegrate in the intestine. Polymeric and other coating materials may be used to modify release. Multiple compressed tablets are made by more than one compression cycle. These include inlay tablets, layered tablets and press-coated tablets. Compressed tablets can be formulated into controlled-release tablets which release drug over a prolonged period of time. Typically, these tablets to provide pulsatile or sustained release.

Compressed tablets can also be formed into dosage forms for purposes other than direct oral delivery. These include, but are not limited to, disintegration into solution, effervescent, chewable or dispersible tablets, compressed suppositories, pessaries or inserts, and buccal and sublingual tablets.

A number of diluents are used in tableting to increase the bulk of the tablet to a practical size for compression. Diluents commonly used include dicalcium phosphate dihydrate, tricalcium phosphate, calcium sulfate, lactose, spray-dried lactose, pregelatinized starch, microcrystalline cellulose, cellulose, kaolin, mannitol, sodium chloride, dry starch and powdered sugar. Certain diluents, particularly mannitol, trehalose, lactose, sorbitol, sucrose and inositol, are used to make chewable tablets. Microcrystalline cellulose is a nonfibrous form of cellulose obtained by spray-drying washed, acid-treated cellulose and is available in several grades that range in average particle size from 20-100µm.

Certain drawbacks are inherent in the use of any diluent and they must be chosen based on the intended use and reactivity with the drug. Lactose is widely used. However the combination of amine bases or amine salts with lactose in the presence of an alkaline lubricant results in losses in the bioavailability of the active agent and tablets that discolour on aging.

Hydrous lactose does not have properties that permit it to flow and use is limited to tablet formulations prepared by the wet granulation method. Both anhydrous lactose and spray-dried lactose have good flow properties eg flowability and compressibility and can be used in direct compression provided a suitable disintegrant and lubricant are present in the tablet. Other constraints to the use of lactose in tableting are discussed below.

Binders and granulators are used to impart cohesive qualities to the powdered material. Binders and granulators impart cohesiveness to the formulation which insures the tablet remaining intact after compression, as well as improving the free-flowing qualities by affording granules of a desired hardness and size.

The selection of a particular formulation of components is determined by a variety of parameters including the physical characteristics required of the finished delivery system. The exact formulation will contain a number of components, each chosen to impart a specific function and together to effect the specific desired properties. These are usually determined empirically.

The physical characteristics of tablets are measured in terms of strength, friability uniformity of dimensions, weight and disintegration or dissolution time.

Tablet strength, also termed hardness or tensile strength, is a measure of the cohesiveness of a tablet.

Hardness is defined as the resistance of the tablet to chipping, abrasion or breakage under conditions of storage, transportation and handling. There are a number of machines manufactured for measuring hardness, such as the Hebelein, distributed by Vector. If a tablet is too hard it may not disintegrate in the required period of time or meet the dissolution specification; if it is too soft it will not withstand the handling during subsequent processing, packaging, film coating, transport etc.

Friability is the ability of a tablet to resist chipping and abrasion. Friability is measured by tumbling tablets and determining the weight loss. Tumbling can be performed manually or mechanically, for instance by a Roche friabilator.

The thickness, weight, disintegration time and content uniformity of a tablet must be relatively invariant from run to run. Tablets may be subject to further processing such as coating prior to packaging. A wide variety of coatings are known in the art.

Trehalose (α-D-glucopyranosyl-(-D-glucopyranoside) is a naturally occurring, non-reducing disaccharide which is available commercially in the dihydrate form.

US-A-4,678,812 and US-A-4,762,857 describe methods and compositions of tableting powders using a process of powder formation referred to as the S-1 process. These patents disclose a process of forming an aqueous mixture or dispersion of all of the components of the finished tablet including trehalose, active ingredients, excipients etc., spraying the aqueous mixture onto the surface of a moving bath of perfluorocarbon liquid, followed by lyophilization of the frozen droplets to dried powders. The S-1 method is used to prevent the formation of amorphous forms of trehalose. The mixing of all the components in aqueous solution prior to formation of the powder is an integral part of the S-1 process invention as it is required to achieve the necessary degree of homogeneity or batch to batch standardization. The S-1 method is described more fully in U.S. Patent Nos. 3,932,943 and 3,721,725. EP-A-606753 and EP-A-636693 disclose use of trehalose as a dessicant in food products, cosmetics and pharmaceuticals.

EP-A-693558 relates to the production of trehalose in its hydrate or anhydrous crystalline form. EP-A-691407 relates to the production of trehalose using a trehalose-releasing enzyme. The production of anhydrous crystalline trehalose is disclosed.

It has now been found that anhydrous amorphous trehalose can be used as an excipient to produce dosage forms of a higher quality than those prepared using lactose, or other carbohydrate excipients without employing the S-1 process or the necessity for combining the components in aqueous solution or aqueous mixture prior to powder formation. Furthermore trehalose does not undergo chemical reactions eg the Maillard reaction with amino, amine or amido groups that occurs with reducing sugars such as lactose. It has also been found that the amorphous form of trehalose is preferred in production of dosage forms of high quality and homogeneity.

According to a first aspect of the present invention a method of making a tablet comprises the steps of:
a) combining in a substantially dry form a therapeutically-effective amount of an active agent with an amount of anhydrous amorphous trehalose, sufficient to act as a diluent ;
b) mixing the dry components of step a) to form substantially homogeneous powders, pellets, granules or microgranules ; and
c) forming a tablet from the product of step b), wherein the tablet comprises anhydrous amorphous trehalose.

The invention encompasses methods of producing tablets using anhydrous amorphous trehalose (AAT) and different physical forms of powdered trehalose. The additional forms of trehalose include, trehalose dihydrate (TD), which is crystalline, amorphous trehalose (AT) which is vitreous, and anhydrous crystalline trehalose (ACT).

"Anhydrous trehalose" refers to any physical form of trehalose containing less than about 2 percent water. The anhydrous forms of trehalose may contain from about 0-2% water and still retain beneficial properties in tableting. Amorphous trehalose (AT) contains about 2-9% water and TD contains about 9-10% water.

The invention encompasses formulations formed with or without a disintegrant. Dosage forms including disintegrants have been found to dissolve rapidly and release the active ingredient into the aqueous medium.

In one method of making tablet formulations, an active agent is incorporated into a solution of trehalose and dried to form a trehalose matrix. The trehalose in the resultant matrix will be initially in the form of AT. This matrix may then be blended with trehalose and any other excipients and formed into dosage forms eg by tableting.

Provided the active agent is heat stable in the presence of trehalose, the AT can be further treated to obtain AAT, ACT or mixtures thereof prior to use in manufacture of dosage forms. The formulations thus obtained are also encompassed by the invention.

Preferred embodiments of this invention include tablets composed of AAT and ACT with or without TD and/or AT optionally including other excipients. Formulations having different proportions or amounts of these components result in dosage forms with a wide variety of properties suitable for use with a number of active agents with different physicochemical and physiological properties.

The invention also includes methods of making tablets with increased dissolution rates. These rapidly soluble formulations are made by combining trehalose powder, active agent, a volatile salt, an amount of a binder sufficient to impart the required hardness to the finished dosage form and any other excipients, and forming dosage forms from the mixture. The formulations are then exposed to a vacuum or heat for a time and under conditions sufficient to remove the volatile salt or a constituent thereof. The dosage forms thus obtained have increased surface area, decreased weight and increased dissolution rate compared to solid tablets of like dimensions. The formulations thus formed are also encompassed by the invention. Use of volatile salts is described in greater detail in copending PCT/GB97/00368 claiming priority from US 08/599277.

Materials commonly used as binders include starch, gelatin and sugars such as sucrose, glucose, dextrose, molasses and lactose. Natural and synthetic gums that are also used may include acacia, sodium alginate, extracts of Irish moss, panwar gum, ghatti gum, mucilage of isapol husks, carboxymethylcellulose etc. Any other suitable binders known in the art may be added including, but not limited to, polyvinyl pyrolidone (PVP) for example Ludipress, Kollidon (trade marks) and HES. Kollidon VA64 (BASF) is a preferred polyvinyl pyrolidone based binder. Ludipress (BASF) is a commercial tableting mixture of lactose and PVP. Byco A (croda) is degraded gelatin of 2500-4000 M. Wt. range. HES (NPBI) is hydroxyethyl starch. As discussed below, under certain conditions, additional binders are necessary to achieve an appropriate degree of hardness in the dosage forms.

According to the present invention a method of producing anhydrous amorphous trehalose comprises the steps of heating trehalose dihydrate at a temperature, for a duration of time and under conditions sufficient to reduce the water content to about 1 to 2%.

The anhydrous trehalose obtained is particularly suitable for use as a tableting excipient. Although any methods known in the art may be used to make the anhydrous trehalose, the present method is preferred. This method may reproducibly provide high quality anhydrous amorphous trehalose powders, simply, economically and without the use of solvents. Preferred methods are fast, simple and do not require the complicated, expensive and time-consuming methods of the prior art. No solvents need be used. Spray drying and freeze drying and the variable results and high energy consumption thereof can be avoided.

The method of making ACT, useful in the invention, includes the steps of heating TD, obtained from any source, at a temperature, for a duration of time and under conditions sufficient to remove the water molecules. The heating is preferably performed under ambient, atmospheric, pressure. Preferably, the heating is at about 50-105°C. More preferably, the heating is at about 60 - 90°C. Most preferably, the heating is at about 70°C. Typically, the preferred heating duration is about 4-96 hours. More preferably, the heating duration is about 12-72 hours. Most preferably, the heating duration of time is about 24 hours. Typically, the TD is heated at 70°C for 12 - 24 hours to produce ACT.

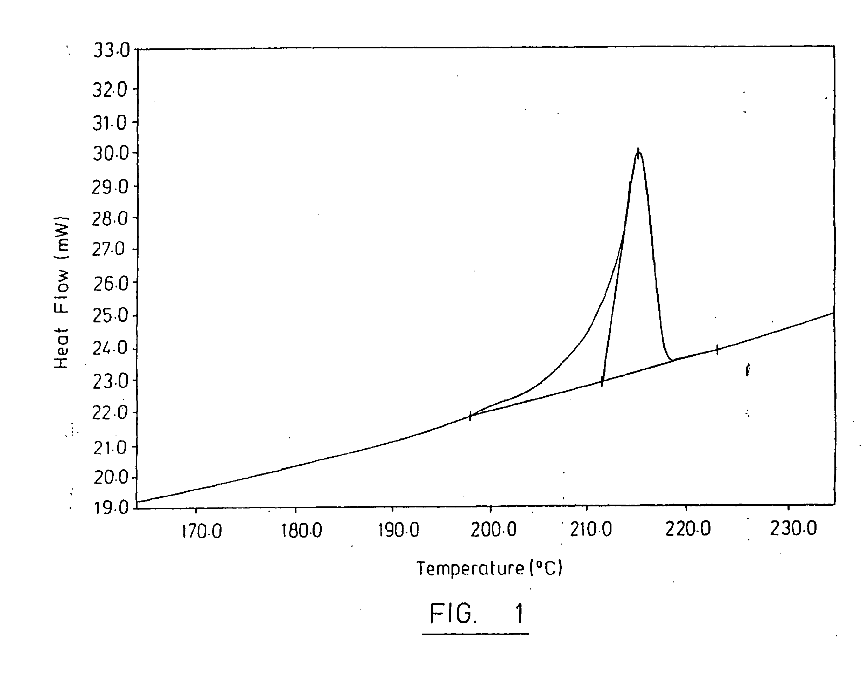

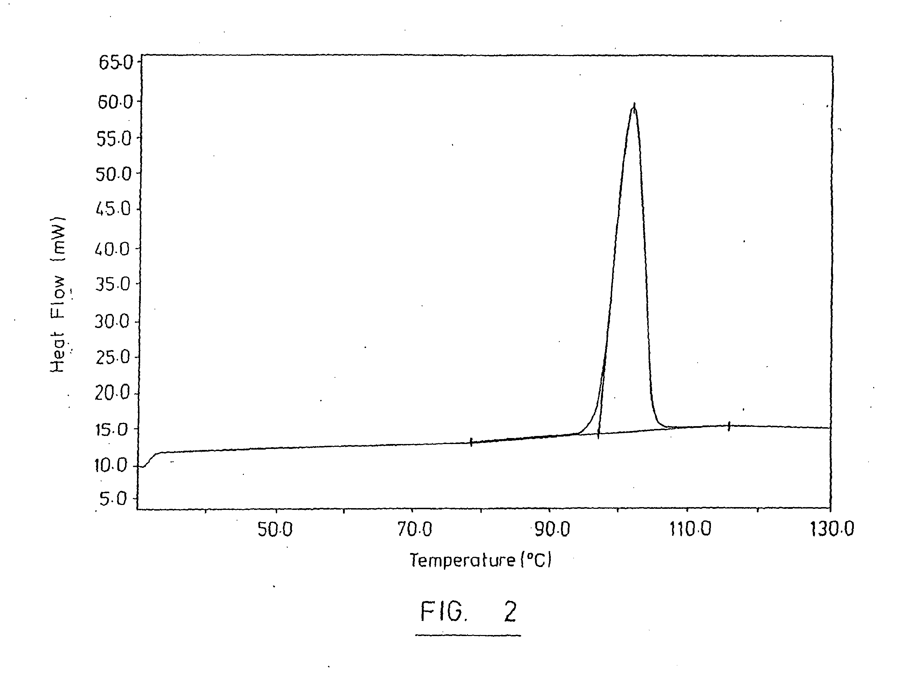

It has now been shown that the method described herein produces ACT. As shown in Figure 1, and described more fully in Example 1a, the DSC analysis of the product obtained by heating TD for 48 hours at 70°C, shows a sharp endothermic peak at about 200°C indicative of the melting of the crystalline form of the anhydrous trehalose powder. For comparison of the ACT to the crystalline TD form, compare Figure 2, which depicts the DSC analysis of crystalline TD, and Figure 1. The DSC trace of Figure 2 also shows the sharp endothermic peak characteristic for the melting of the crystalline form, but at the much lower temperature of about 96°C, the specific melting point of crystalline TD.

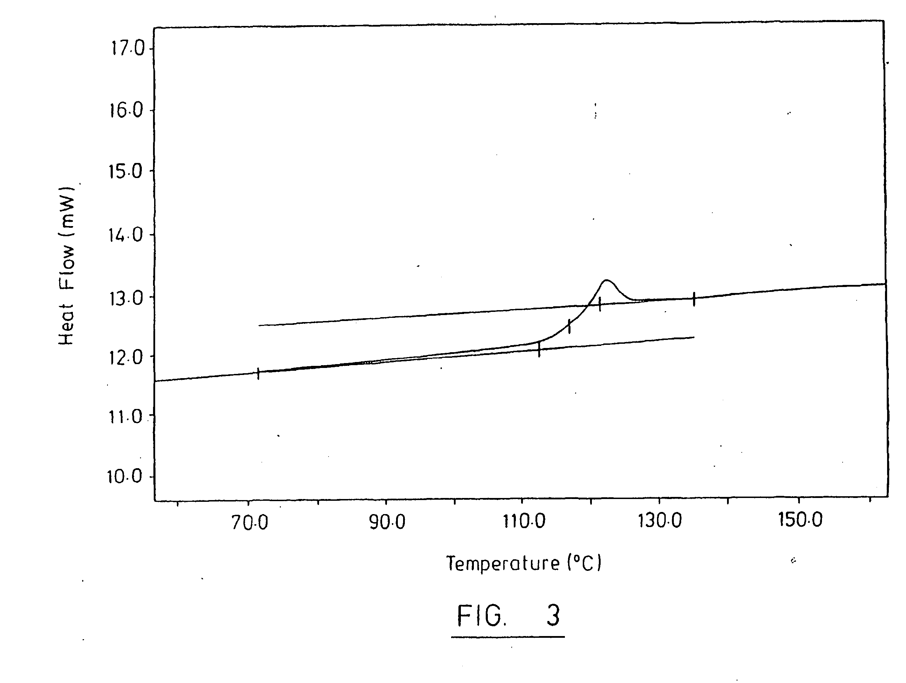

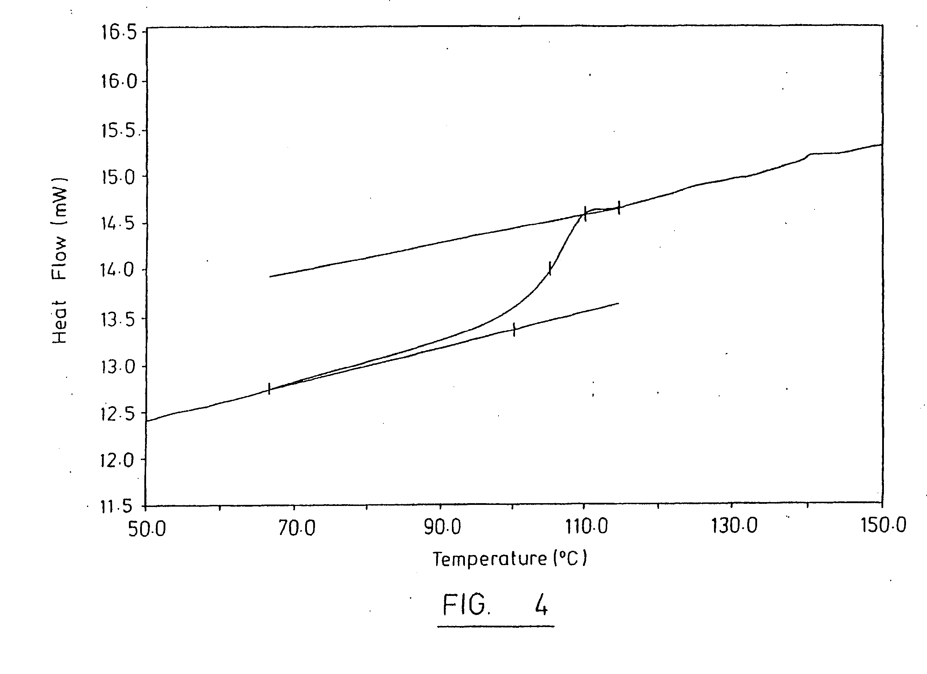

Applying reduced pressure during the heating of TD results in the formation of AAT. Typically, the preferred reduced pressure is about 1-30,000 mTorr. More preferably, the reduced pressure is about 10-10,000 mTorr. Most preferably, the reduced pressure of is about 750 mTorr. Typically, the sample is heated under reduced pressure for 18 - 24 hours. Figure 3 shows the DSC analysis of AAT obtained by heating crystalline TD at 70°C for 24 hours and at 1.5 Torr. This is described more fully in Example 1b. Note that the trace obtained does not have the single distinct endothermic peak characteristic of crystalline trehalose, but shows the glass transition typical of amorphous matrices. Figures 4-6 show the DSC traces of AT produced by FTGs, spray drying and freeze drying of trehalose solutions containing active agents. The glass transition temperatures (Tg) seen are dependent on the residual water contents of the AT matrix with increased water contents corresponding to lower measured Tgs. These are described more fully in Examples 1c-1f.

The anhydrous trehalose formulation may be produced by making a foamed trehalose glass containing the active agent followed by the powdering the mixture. The preparation of trehalose glasses is disclosed in our copending PCT/GB96/01367.

Water may be removed from hydrated trehalose either before or after application of heat. Removal of water before heating affords ACT whereas heating before or during removal of water affords AAT. While not being bound by theory this may be due to removal of water from the crystalline dihydrate either before or after melting the TD. Thus in a preferred method of preparation of AAT, hydrated trehalose is placed under a vacuum and heated to 80°C.

Use of a mixture of AAT and ACT in formulations is preferred. The crystalline material has good flow properties and excellent water absorption.

The two anhydrous forms of trehalose may be mixed in a wide variety of ratios to obtain tablets of varying properties. TD may also be incorporated to impart desired characteristics of hardness, friability and dissolution times. The anhydrous trehalose mixtures can be obtained according to the methods described herein or by any other method known in the art. The components, anhydrous trehalose, TD, active agent, other binders and excipients, if any, are combined, mixed, and formulated into delivery systems by any method known in the art. Suitable methods include, but are not limited to, milling, dry granulation, pelletisation, direct compression, roller compaction, and spheronization. Detailed descriptions of tableting methods are provided, for instance in Remington: The Science and Practice of Pharmacy, 19th ed. Vol. II (1995) Mack Publishing Co., Pennsylvania.

Alternatively, the active agent can be mixed with trehalose, dried and then subjected to a combination of heat and vacuum which yields a desired mixture of crystalline and AAT. The proper combination of heat and vacuum can be determined empirically and depends on the amount and type of active agent and the ratio of amorphous to ACT desired. Applying only heat results in purely ACT whereas heat and the upper ranges of vacuum results in only AAT. By varying the amount of vacuum applied, ACT concentration can be controlled. The ratio of the various physical forms can be determined for instance by DSC. The optimal mixture will be determined empirically in practice by determining which processing conditions yield delivery systems with the most desirable characteristics.

In one method of compressed tablet formation, the dry components are mixed thoroughly, treated to produce a more homogeneous mixture and then compressed into tablets. The mixture may be compressed into slugs and slugs ground and screened to 14 to 16-mesh granules. The granules may be recompressed into the final tablet.

In addition to producing dosage forms or other formulations of superior physical properties,

AAT provides increased stability of the active agent. Anhydrous amorphous trehalose further provides protection of the active agent from ambient humidity.

Without being bound by theory, applicants believe that the protection from humidity offered by anhydrous amorphous trehalose may be due to its absorption of water molecules to produce TD and that this sequestration of the atmospheric water molecules from the active agents decreases the exposure of the active agent to moisture. In addition, trehalose is nonreducing and does not undergo the Maillard reaction in the presence of amino groups and thus is suitable for use with active agents containing labile amino groups.

The use of anhydrous amorphous trehalose as a diluent in tablets imparts improved physical properties compared to prior art diluents. For instance, the active agents may be more stable. The tablets may be more resistant to humidity, and volatile salts can be added to the tablet and completely removed after tableting to yield porous tablets that dissolve rapidly upon contact with aqueous solvent humidity. In the following examples, anhydrous amorphous trehalose shows clear superiority over TD in maintaining the stability of an active agent.

The invention further encompasses methods of forming tablets wherein volatile salts are added during formulation and completely removed after formulation to yield porous tablets or other dosage forms. These rapidly soluble (RS) dosage forms dissolve rapidly upon contact with aqueous solvents.

Although singular forms may be used herein, more than one active agent and more than one excipient may be present in the compositions described and claimed. Determination of the types and effective amounts of these components is within the skill of one in the art.

A wide variety of active agents may be used in formulations forms of this invention, examples include: antiinflammatory drugs, analgesics, antiarthritic drugs, antispasmodics, antidepressants, antipsychotics, tranquilizers, antianxiety drugs, narcotic antagonists, antiparkinsonism agents, cholinergic agonists, chemotherapeutic drugs, immunosuppressive agents, antiviral agents, antibiotic agents, appetite suppressants, antiemetics, anticholinergics, antihistaminics, antimigraine agents, coronary, cerebral or peripheral vasodilators, hormonal agents, contraceptives, antithrombotic agents, diuretics, antihypertensive agents and cardiovascular drugs

The invention is further described by means of example but not in any limitative sense with reference to the accompanying drawings of which:
Figure 1 is a graph depicting the differential scanning calorimetry (DSC) trace of the crystalline form of anhydrous trehalose (ACT) formed by the removal of water from the crystalline dihydrate (TD). In each of the Figures depicting DSC traces, the temperature was raised in increments of 10.0°C/min. The profile shows a characteristic crystalline melt endotherm. In Figure 1, the melting point is 215°C.
Figure 2 is a graph depicting the DSC trace of TD, the profile obtained is indicative of crystalline TD with the characteristic melt endotherm at a lower temperature. In Figure 2, the melting point is 101.5°C.
Figure 3 is a graph depicting the DSC trace of the amorphous form of anhydrous trehalose (AAT) formed by the removal of water from the crystalline dihydrate (TD) under vacuum. In Figure 3, the glass transition temperature, Tg, is 117°C.
Figure 4 is a graph depicting the DSC trace of the amorphous form of anhydrous trehalose (AAT) formed by quenching from the melt. In Figure 4 the Tg, is 105°C.
Figure 5 is a graph depicting the DSC trace of AT formed by freeze-drying from solution. In Figure 5, Tg is 100°C.
Figure 6 is a graph depicting the DSC trace of the amorphous form of trehalose (AT)AT formed by spray-drying from solution. In Figure 6 Tg is 69°C.
Figure 7 is a graph depicting the DSC trace of AT formed by vacuum AT formed by vacuum-drying from solution to produce a foamed trehalose glass (FTG). In Figure 7 Tg is 84°C.

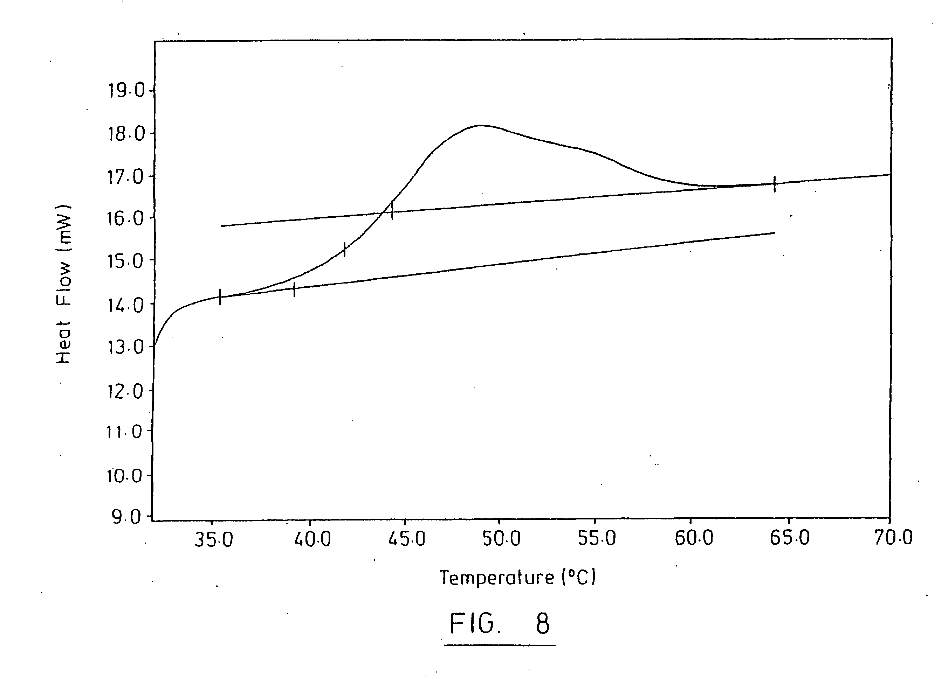

Figure 8 is a graph depicting the DSC trace of the amorphous form of anhydrous trehalose (AT) containing active peptide in a foamed trehalose glass (FTG)FTG. In Figure 8 Tg is 42°C.
Figure 9 is a graph depicting the DSC trace of the FTG containing active peptide (sample as in Figure 8) after storage at ambient humidity depicting the melting of dihydrate (TD) formed due to devitrification on exposure to the atmosphere. In Figure 9, the melting point is 100°C.

For the purposes of the following examples, particle size was not stringently controlled, but powders were formulated to have flow characteristics suitable for dosage form production. The flow characteristics obtained were found to enable loading the tablet die without difficulty. A sieving procedure can be incorporated to ensure a more even particle size. This is essential in larger batches to guarantee thorough mixing of the components and it is well within the skill of one in the art to devise a suitable sieving procedure.

In the examples presented herein, magnesium stearate was routinely used as a lubricant, and is the preferred lubricant. Any other suitable lubricant may be used including, but not limited to, talc, calcium stearate, stearic acid, hydrogenated vegetable oil, Lutrol (trade mark) and polyethylene glycol (PEG). Disintegrants are added to facilitate breakup or disintegration of the tablet before or after administration. Colouring agents make the dosage form more aesthetic in appearance and may serve as identification. Flavouring agents are usually added to provide sweetness to chewable or dissolvable tablets. The invention encompasses tablets formed from trehalose with or without any excipient or combinations of excipients.

### EXAMPLE 1

### Methods of making AAT and ACT

### 1a. Formation of ACT

Crystalline TD was incubated in open trays at temperatures of 55°C, 70°C and 80°C in a standard laboratory oven for 24-72 hours. Samples were assayed for water content by Karl Fischer analysis and selected samples were also analysed by DSC. Surprisingly, the samples all showed water contents ranging from 0.1 - 2%, even those heated at just 55°C. Figure 1 shows the DSC trace of a sample heated at 70°C for 48 hours. The water content of the sample was 0.33%. The sample showed a crystalline melt at approximately 210 - 216°C characteristic of the melting temperature of ACT. This endotherm is distinct from the melt endotherm at the lower temperature of 96 - 101°C characteristic of TD seen in the DSC analysis of crystalline dihydrate as shown in Figure 2.

### 1b. Formation of AAT

Crystalline TD was incubated in open trays for 16 - 24 hours in either a Heraus vacuum oven with a reduced pressure of 1.5 Torr, or in a FTS freeze drier with a reduced pressure of 30 mTorr, and the shelf temperature set at 60°C. Samples were again assayed for water content by Karl Fischer analysis and selected samples were also analysed by DSC. Samples typically showed water contents lower than those in Example 1a, ranging from 0.1 - 1.5%. Figure 3 shows the DSC trace of a sample heated at 60°C for 24 hours in a vacuum oven at 1.5 Torr. The water content of the sample was below 0.1%. Surprisingly, the sample no longer showed a crystalline melt at approximately 215°C, but instead showed a glass transition of 116 - 117°C characteristic of the amorphous form of trehalose demonstrating the formation of AAT.

### 1c. Formation of AAT by quenching from the melt

Crystalline TD was heated at 96 - 100°C with a minimum quantity of water to produce an isotropic melt which was transferred to an oven at 185°C for a few minutes before cooling the melt to produce a clear trehalose glass. The glass was then ground in a Waring blender to give a fine powder. Samples were again assayed for water content by Karl Fischer analysis and selected samples were also analysed by DSC. Samples typically showed water contents ranging from 0.1 - 2%. Figure 4 shows the DSC trace of a typical sample having a glass transition of 100 - 102°C characteristic of the amorphous form of trehalose again demonstrating the formation of AAT.

### 1d. Formation of AAT containing active agent by freeze drying

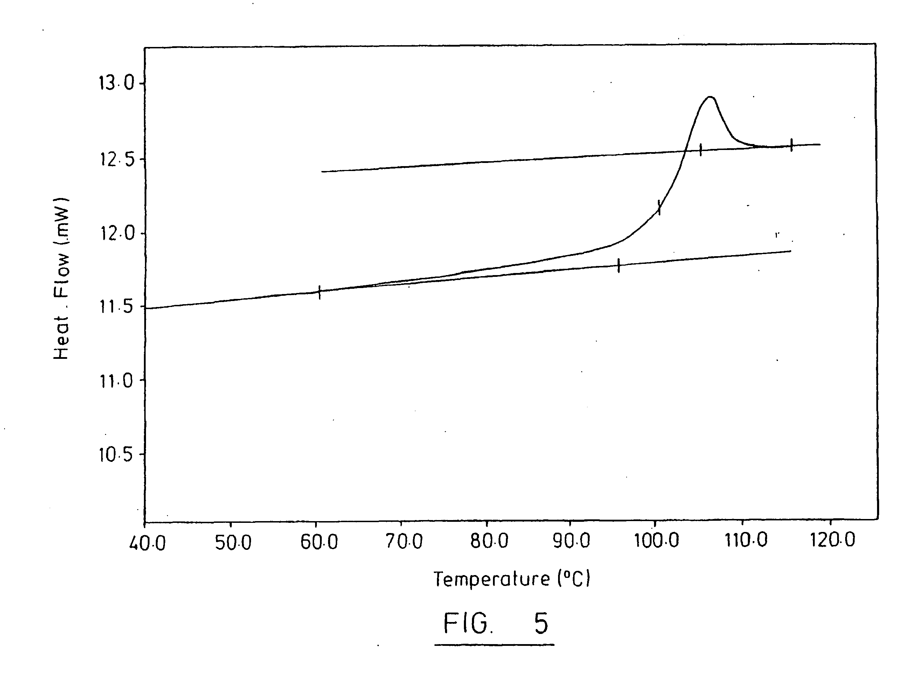

Samples containing a solution of 20% (w/v) trehalose were frozen at 5°C/hr to -40°C and dried under vacuum of 30 mTorr at a shelf temperature of -35°C for 40 hrs, followed by a further 2 hrs drying at the raised shelf temperature of 25°C in an FTS freeze-drier. A dry powder typical of freeze drying was obtained. Samples typically showed water contents ranging from 0.1 - 2%. Figure 5 shows the DSC trace of a typical sample with water content of 0.79%, showing a glass transition of 100 - 101°C characteristic of the amorphous form of trehalose again demonstrating the formation of AAT.

### 1e. Formation of AAT containing active agent by spray drying

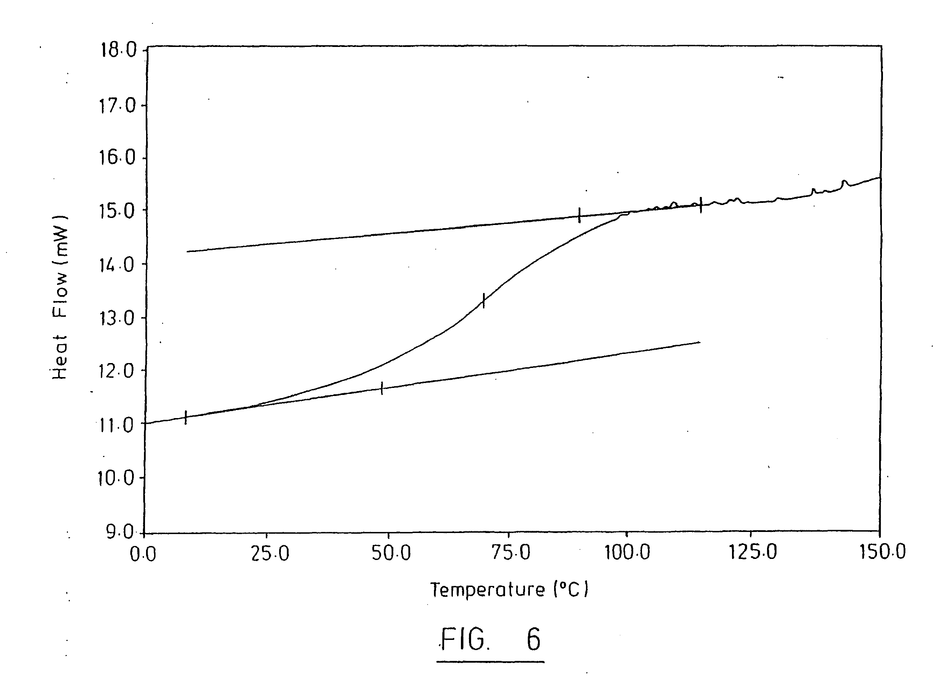

Samples containing a solution of 20% (w/v) trehalose were spray dried in a lab-scale Buchi spray drier at a range of inlet temperature of 150 - 180°C at a flow rate of 10% of the pump capacity. Samples typically showed water contents higher than in Examples 1a-d ranging from 0.5 - 5%. Figure 6 shows the DSC trace of a typical sample with water content of 4.5%, showing a glass transition of 65 - 70°C characteristic of the amorphous form of trehalose. Thus this method produces AAT suitable for incorporation into tablets in combination with anhydrous trehalose and TD.

### 1f. Formation of AAT containing an active agent in FTG

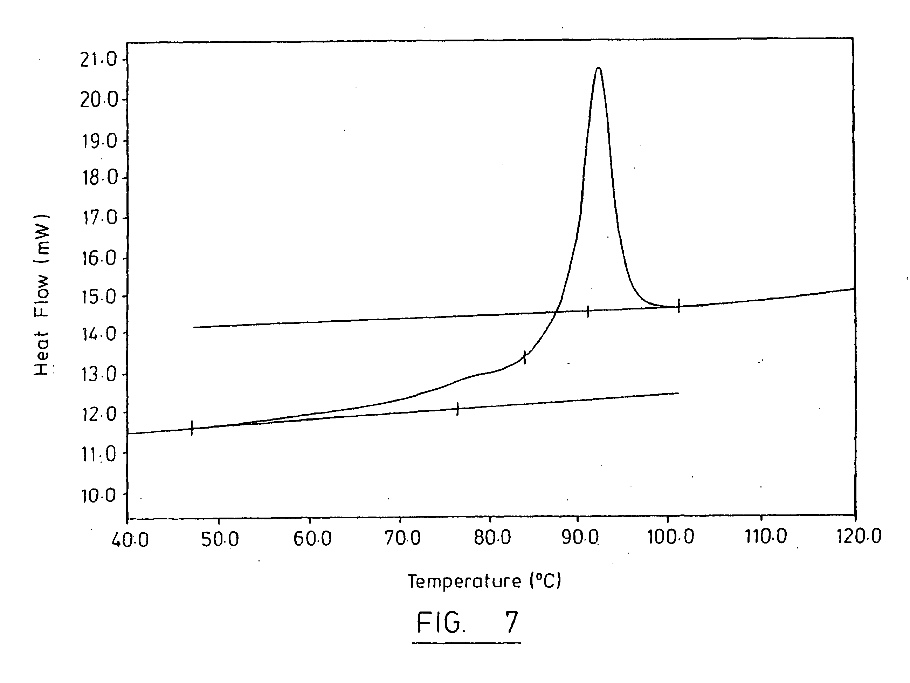

300 µm aliquots of a solution of 43.4 mg/ml trehalose containing 66 mg/ml of an antimicrobial peptide was dried in 10 ml polypropylene tubes (10 mm diameter) in the FTS drier. Samples, at 25°C, were loaded onto a shelf in the drier that had been preheated to 35°C. The vacuum pressure in the chamber was progressively reduced to 20 Torr over 10 minutes. This pressure was held for a further 30 minutes before the pressure was further reduced to 30 mTorr. After 17 hours, the shelf temperature was increased to 50°C. This shelf temperature was maintained for 3 hours after which the cycle was stopped. The FTGs produced have an open plug-like structure similar to freeze-dried materials. Moisture content was typically 1.1 to 2% (w/w). Figure 7 shows the DSC trace of a typical sample with water content of 1.59% showing a glass transition of 83 - 84°C characteristic of the amorphous form of trehalose.

### EXAMPLE 2

### Use of AAT and ACT in tablet production

The following example utilizes the amorphous and crystalline forms of anhydrous trehalose, for the laboratory scale production of tablets. The anhydrous trehalose was manufactured by heating crystalline TD at 60°C, at atmospheric pressures to obtain ACT or under vacuum with heat to obtain the AAT as described in Examples 1a and 1b, respectively.

The blends used in the tableting of anhydrous trehalose contained either single forms of anhydrous trehalose or mixtures thereof, and also optionally contained a number of commercially used binders such as Kollidon VA64, Ludipress, BycoA and HES and lubricants such as magnesium stearate, sodium lauryl sulfate and Lutrol.

The production of rapidly dissolving tablets was again achieved by the addition of a volatile salt to the tableting blend followed by the removal of the salt under vacuum to obtain a porous tablet that showed increased dissolution rates compared to tablets of the same blends without the volatile salt incorporated. Aliquots of 0.5 g of powder were weighed out into a 0.5 inch Manesty type die. The tablet was formed by hammering onto the upper punch (A single light positioning blow, followed by a stronger single blow). The tablets were of a convex oval shape and at least 3 mm thick. On release from the die, the tablets were stored in sealed vials. In tablets formed from blends containing ammonium bicarbonate, the volatile salt was removed under a vacuum of 1.5 Torr at 60°C for six hours to yield porous, rapidly dissolving tablets which were again stored in sealed vials prior to analysis. Disintegration and dissolution of the tablets was studied in distilled water at 28°C with gentle agitation.

The results obtained on tableting the various blends of anhydrous trehalose and disintegration and dissolution are presented in Tables 1 and 2 respectively. In Table 1, * stands for anhydrous trehalose, K stands for Kollidon and B stands for BycoA. Table 2 shows the effect of increased porosity on rate of disintegration/dissolution of selected tablets. Similar results were obtained for the formation of tablets from these formulations using the automated Manesty F3 tableting press.

**TABLE 1**

| SAMPLE | COMPOSITION (Wt%) | | | | COMMENTS |
|---|---|---|---|---|---|
| | Trehalose form | Tab.aid | Amm. bicarb | Mg Stearate | |
| 1 | 99.5 ACT | - | | 0.5 | Good |
| 2 | 64.97 ACT | HES 4.8 | 29.6 | 0.53 | Excellent |
| 5 | 99.5 AAT | - | - | 0.5 | Good |
| 6 | 69.5 AAT | - | 30 | 0.5 | Good |
| 7 | 59.5* | - | 40 | 0.5 | Some lamination |
| 8 | 49.5 | - | 50 | 0.5 | Occasional lamination |
| 14 | 39.5* | B 10 | 50 | 0.5 | Mostly good |
| 15 | 44.5%* | B 5 | 50 | 0.5 | Good. Few laminate after storage |
| 18 | 69.5* | - | 30 | 0.5 | Excellent |
| 19 | 67.5 | B 2 | 30 | 0.5 | Good |
| 22 | 50* | - | 50 | - | Excellent |
| 25 | 55* | K 5 | 40 | - | Excellent |
| 27 | 54.5* | K 5 | 40 | 0.5 | Excellent |

**TABLE 2**

| SAMPLE | DISINT | DISSOL | COMMENTS |
|---|---|---|---|
| 1 | 22 secs | 2.5 mins | - |
| 2 | - | 7.5 mins | no disintegration |
| 14 | - | 1 min | - |
| 15 | 15 secs | 45 secs | most dissolves in 30 secs |
| 18 | - | 4 mins | no disintegration |
| 19 | - | 2 mins | no disintegration |
| 22 | 10 secs | 45 secs | - |
| 25 | 15 secs | 1 min | - |
| 27 | 10 secs | 1 min | - |

The results presented in Table 2 indicated that removal of volatile salt to give porous tablets significantly increased the disintegration and dissolution rates of the tablets produced. Complete removal of the volatile salt was assessed by difference in tablet weight before and after vacuum treatment. AT compresses better than the FTG and volatile salt can be incorporated in up to at least 50 weight %. This leads to a highly porous matrix after the volatile salt has been removed. AT alone remains a good binder, though some loss in intrinsic strength is seen n tablets of blends incorporating ammonium bicarbonate and especially once the volatile salt has been removed. Without a binder of some kind, these porous tablets are very fragile and a balance is therefore essential between a high proportion of volatile and inclusion of a small percentage of binder. These porous tablets dissolve rapidly when compared to tablets formed from trehalose alone; the time for full dissolution is generally reduced from 10 - 15 minutes down to less than 1 minute.

### EXAMPLE 3

### Use of AAT for making tablets containing active agent: Stability enhancement over use of crystalline TD

Pre-formulated powders, containing AAT and a synthetic vasoactive peptide, together with binders and lubricants such as Kollidon VA64 (FASF), Citric Acid, Aerosil 200 (Degussa), Magnesium stearate (BDH), Sodium lauryl sulfate (BDH), Polyethylene glycol 8000 (BDH), Glyceryl monostearate (Akzo Nobel) and Lutrol F68 (BASF) were blended using a Braun coffee grinder for a few seconds, before being sieved through a 30 mesh (500 micron) screen. Tableting was performed on a Manesty F3 single station, direct compression press. The speed was set at about 60 - 70 tablets per minute and compression was approximately 1 - 1.5 tons. The flow of the blend was controlled to give the desired weight distribution of the tablets produced.

The general appearance of the tablets was recorded. Hardness was measured on a Schleuniger-4M hardness tester. Friability of the tablets was estimated by vigorously shaking a number of tablets together and visually inspecting for fragmentation. Dissolution was tested using 5 tablets, suspended in aluminum baskets, in 11 of stirred (500 rpm speed) deionised, distilled water at 37°C. The dissolution time recorded, was that when all the soluble excipients had disappeared from view. Hygroscopicity of the tablets was assayed by weight change after 24 hours incubation at several relative humidities. Stability of the vasoactive peptide was assayed by HPLC analysis. Tables 3 - 5 illustrate selected formulations that were tableted and tested as above. Table 3 shows the compositions of active agent incorporated in the FTG tableted with AAT as the tableting excipient. Table 4 shows the incorporation of active agent directly into the AAT tablet by simply mixing the active agent with AAT to yield the tableting blend. Table 5 shows the effect of conversion of AAT to dihydrate on tableting of blend.

**TABLE 3**

| No. | % | Mg/ tablet | Raw Material | Tot.Qty (gms) |
|---|---|---|---|---|
| 1 | 95.42 | | AAT + 10% FTG containing active peptide at 1.11 mg/g | 171.76 19.08 |
| 2 | 0.16 | | Citric acid | 0.32 |
| 3 | 4.0 | | Kollidon VA64 | 8.0 |
| 4 | 0.02 | | Aerosil 200 | 0.04 |
| 5 | 0.4 | | Magnesium stearate | 0.8 |
| Total | 100 | 50 | | 200 |

The results presented in Table 3 indicate that excellent tablets were formed. The tablets had good binding properties and little excess dust was produced. The dosing of the die was very reproducible and therefore consistent weight was achieved.

**TABLE 4**

| No. | % | Mg/ tablet | Raw Material | Tot. Qty (gms) |
|---|---|---|---|---|
| 1 | 95.4 | | AAT | 190.80 |
| 2 | 0.0185 | | Vasoactive peptide | 0.0370 |
| 3 | 0.16 | | Citric acid | 0.32 |
| 4 | 4.0 | | Kollidon VA64 | 8.0 |
| 5 | 0.02 | | Aerosil 200 | 0.04 |
| 6 | 0.4 | | Magnesium stearate | 0.8 |
| Total | 100 | 50 | | 200 |

The results presented in Table 4 indicate that excellent tablets were formed. The dosing was again consistent. Good binding properties were observed with the use of Kollidon.

**TABLE 5**

| No. | % | Mg/ tablet | Raw Material | Tot. Qty (gms) |
|---|---|---|---|---|
| 1 | 93.0 | | AAT + 10% FTG (blank preparation) | 167.40 18.60 |
| 2 | 2.0 | | Lutrol F68 | 4.0 |
| 3 | 4.48 | | Kollidon VA64 | 8.96 |
| 4 | 0.02 | | Aerosil 200 | 0.04 |
| 5 | 0.5 | | Magnesium stearate | 1.0 |
| Total | 100 | 50 | | 200 |

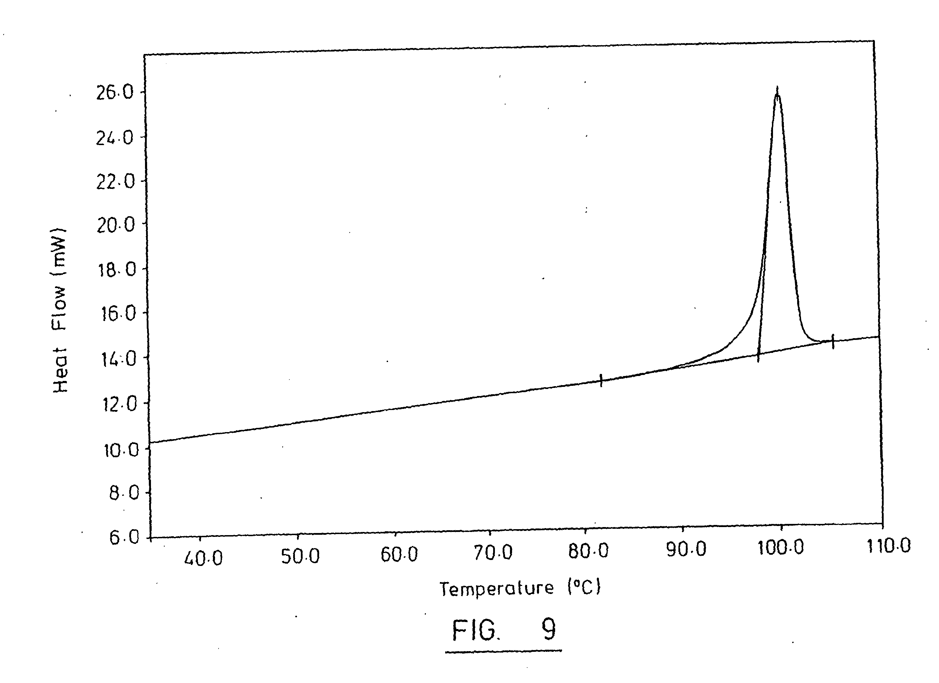

The results presented in Table 5 indicate that excellent tablets were formed when the above blend was used and hard tablets were produced with a high sheen. However, after exposure of the blend to ambient humidities for 48 hours, the tablets formed showed marked lamination. Exposure of the blend to ambient humidity resulted in the conversion of the AAT to the crystalline dihydrate as demonstrated by the DSC analysis shown in Figure 9.

Without exception, excellent tablets were produced with AAT. A mixture of lubricants was found to be most effective in the formulation and a binder generally gave tablets with a higher sheen. Dissolution times were all within the specified 5 minutes. Table 6 shows the measures of physical hardness and dissolution times of the tablets formed using the blends in Tables 3 - 5.

**TABLE 6**

| BATCH REF | HARDNESS (KP) | AVERAGE WEIGHT (mg) | DISSOLUTION TIME (mins) |
|---|---|---|---|
| Blend in Table 1 | 1.0 - 1.2 | 50 | 1.75 - 2 |
| " " " " | 1.4 | 54 | 2 |
| Blend in Table 2 | 1.5 | 52 | 2.75 |
| " " " " | 1.5 - 1.7 | 50 | 2.5 |
| Blend in Table 3 | 2.2 - 2.3 | 51 | 2.25 |
| " " " " | 1.2 - 1.4 | 50 | 2 |

Table 7 shows data for moisture absorption by AAT tablets after 24 hours and 96 hours exposed to specific relative humidities. The selective adsorption of the water taken up by the anhydrous trehalose results in the partitioning of water by the tableting excipient thus protecting the active at the different RHs.

**TABLE 7**

| CONDITIONS | WEIGHT % CHANGE 24 HOURS 96 HOURS | APPEARANCE |
|---|---|---|
| Atmos. 45% R.H | +8.24 No | no change |
| 75% R.H | +8.60 further | no change |
| Atmos 45% R.H | +8.10 change | no change |
| 75% R.H | +8.40 observed | no change |

The weight % change was measured based on the original weight of ten tablets. The results indicate that the tablets adsorb moisture to around 8 - 9% by weight with a devitrification of the AAT to give crystalline dihydrate tablets. Table 8 shows the dissolution of trehalose tablets when exposed to 45% R.H for 96 hours. Table 9 shows the enhanced stability of active agent in tablets using AT over crystalline TD as a tableting excipient. Though the latter show more rapid disintegration and dissolution rates (Table 8), the stability of the active agent may be compromised as shown by the loss in activity observed on storage (Table 9).

**TABLE 8**

| No. | Run No | Exposed to 45% R.H | Dissolution time |
|---|---|---|---|
| 1 | 2 | yes | <30 seconds immediate disintegration |
| 2 | 2 | no | 2 minutes no disintegration |
| 3 | 3 | yes | <30 seconds immediate disintegration |
| 4 | 3 | no | 2.25 minutes no disintegration |

**TABLE 9**

| FORMULATION | INITIAL | 1 WEEK 70°C | 2 WEEKS 70°C |
|---|---|---|---|
| AAT | 100 | 97 | 89 |
| AAT | 100 | 89 | 88 |
| TD | 100 | 42 | 43 |

In conclusion, AT (in various blends) was successfully tableted using a commercial singe stage press. The tablets produced had hardnesses between 1 and 2.3 KP and friabilities <1% and no capping or lamination was seen. The tablets all dissolved within 3 minutes.

The tablets containing active agent formed using anhydrous trehalose as a tableting excipient showed enhanced stability of the active agent compared to tablets formed using the crystalline dihydrate. This may be particularly important for the tableting of labile active agents.

### EXAMPLE 4

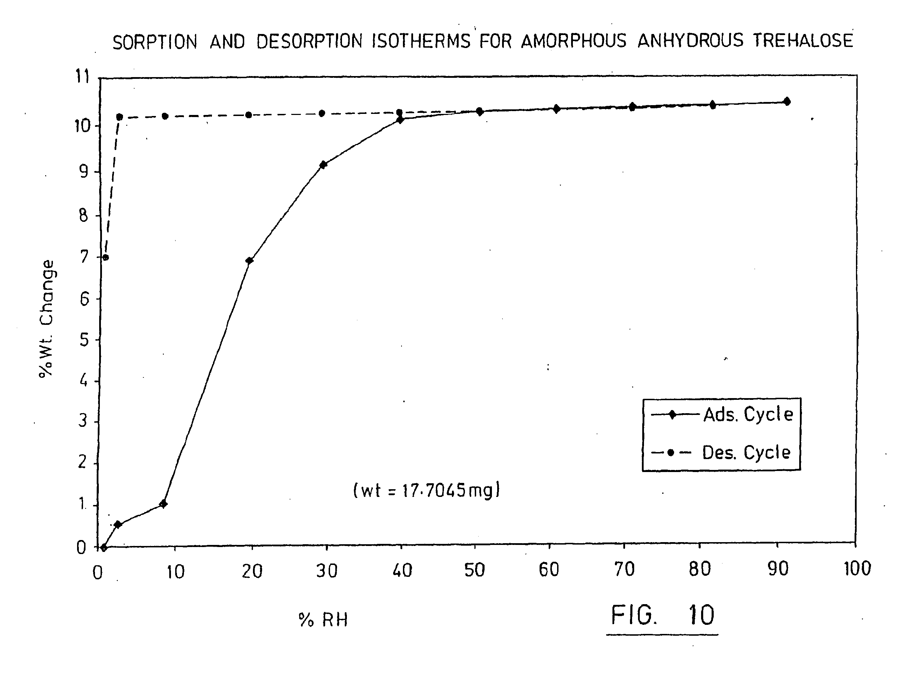

Samples of AAT, ACT and TCD prepared as in Examples 1(a & b) were loaded onto quartz pans of an environmental controlled gas flow microbalance (Dynamic Vapour Sorption (DVS), Surface Measurement Systems, UK) and the sample equilibrated to 0% relative humidity (RH). For the sorption analysis, the RH was then increased in 10% RH steps allowing the samples to equilibrate at each RH. For the desorption analysis, the RH was decreased in 10% RH steps allowing the samples to equilibrate at each RH. The sorption/desorption profile of AAT at 25°C is shown in Figure 10. The sample showed no evidence of recrystallisation during the sorption or desorption period examined. Significantly different profiles were obtained for ACT and TCD with the former showing only minimal sorption below 20% RH and the latter showing only minimal sorption below 50% RH.

## Claims

1. A method of making a tablet, comprising the steps of:
a) combining in substantially dry form a therapeutically-effective amount of an active agent with an amount of anhydrous amorphous trehalose, sufficient to act as a diluent;
b) mixing the dry components of step a) to form substantially homogeneous powders, pellets, granules or microgranules; and
c) forming a tablet from the product of step b), wherein the tablet comprises anhydrous amorphous trehalose.

2. A method according to claim 1, wherein step (a) further comprises combining at least one excipient selected from binders, lubricants, disintegrants, colouring agents and flavouring agents.

3. A method according to claim 2, wherein the excipient comprises a binder selected from starch, gelatin, sugars, natural and synthetic gums, polyvinyl pyrrolidone, hydroxyethyl starch and mixtures thereof.

4. A method according to claim 2 or claim 3, wherein the excipient comprises a lubricant selected from talc, calcium stearate, sodium stearyl fumarate, stearic acid, hydrogenated vegetable oil, polyethylene glycol, magnesium stearate and mixtures thereof.

5. A method according to any preceding claim, wherein the active agent is selected from antipyretics, antiinflammatory drugs, analgesics, antiarthritic drugs, antispasmodics, antidepressants, antipsychotics, tranquilizers, antianxiety drugs, narcotic antagonists, anti-Parkinsonism agents, cholinergic agonists, chemotherapeutic drugs, immuno-suppressive agents, antiviral agents, antibiotic agents, parasiticides, appetite suppressants, antimetics, anticholinergics, antihistaminics, antimigraine agents, coronary vasodilators, cerebral vasodilators, peripheral vasodilators, hormonal agents, contraceptives, antithrombotic agents, diuretics, antihypertensive agents, cardiovascular drugs, opioids and vitamins.

6. A method according to any preceding claim, wherein the diluent further comprises anhydrous crystalline trehalose.

7. A method according to claim 6, wherein the anhydrous trehalose is produced by heating trehalose dihydrate under conditions sufficient to reduce the water content to 1% to 2%.

8. A method according to claim 7, wherein the heating is conducted at a pressure of 0.1333 - 3999.671 Pa (1-30 000 mTorr) to yield anhydrous amorphous trehalose.

9. A method according to claim 7 or claim 8, wherein the heating is at 50-105°C.

10. A method according to claim 9, wherein the heating is at 60-90°C.

11. A method according to claim 10, wherein the heating is at 70°C.

12. A method according to any preceding claim, wherein the tablet further comprises trehalose dihydrate.

## Patentansprüche

1. Verfahren zur Tablettenherstellung, das die folgenden Schritte umfasst:
a) Kombinieren, in einer im Wesentlichen trockenen Form, einer therapeutisch wirksamen Menge eines Wirkstoffs mit einer Menge wasserfreier amorpher Trehalose, die zum Wirken als ein Verdünnungsmittel ausreicht;
b) Mischen der Trockenkomponenten von Schritt a) zum Bilden von im Wesentlichen homogenen Pulvern, Pellets, Granulaten oder Mikrogranulaten; und
c) Formen einer Tablette aus dem Produkt von Schritt b), worin die Tablette wasserfreie amorphe Trehalose umfasst.

2. Verfahren nach Anspruch 1, worin Schritt (a), ferner das Kombinieren von mindestens einem Hilfsstoff umfasst, der aus Bindemitteln, Gleitmitteln, Zerfallsmitteln, Farbstoffen und Geschmacksstoffen ausgewählt ist.

3. Verfahren nach Anspruch 2, worin der Hilfsstoff ein Bindemittel umfasst, das aus Stärke, Gelatine, Zuckern, natürlichen und synthetischen Gummen, Polyvinylpyrrolidon, Hydroxyethylstärke und Gemischen davon ausgewählt ist.

4. Verfahren nach Anspruch 2 oder 3, worin der Hilfsstoff ein Gleitmittel umfasst, das aus Talcum, Calciumstearat, Natriumstearylfumarat, Stearinsäure, hydriertem Pflanzenöl, Polyethylenglykol, Magnesiumstearat und Gemischen davon ausgewählt ist.

5. Verfahren nach einem der vorangehenden Ansprüche, worin der Wirkstoff ausgewählt ist aus: Antipyretika, antiinflammatorischen Arzneimitteln, Analgetika, Antiarthrotika, Antispasmodika, Antidepressiva, Antipsychotika, Tranquillanzien, Anxiolytika, Narkotika-Antagonisten, Antiparkinsonmitteln, cholinergen Agonisten, Chemotherapeutika, Immunsuppressiva, Antivirusmitteln, Antibiotika, Parasitiziden, Appetitzüglern, Antiemetika, Anticholinergika, Antihistaminika, Antimigränemitteln, koronaren Vasodilatatoren, zerebralen Vasodilatatoren, peripheren Vasodilatatoren, hormonalen Mitteln, Kontrazeptiva, Antithrombotika, Diuretika, Antihypertonika, kardiovaskulären Arzneimitteln, Opioiden und Vitaminen.

6. Verfahren nach einem der vorangehenden Ansprüche, worin das Verdünnungsmittel ferner wasserfreie kristalline Trehalose umfasst.

7. Verfahren nach Anspruch 6, worin die wasserfreie Trehalose durch Erhitzen von Trehalosedihydrat unter Bedingungen hergestellt wird, die zum Reduzieren des Wassergehalts auf 1 % bis 2 % ausreichen.

8. Verfahren nach Anspruch 7, worin das Erhitzen bei einem Druck von 0,1333 - 3999,671 Pa (1 - 30 000 mTorr) zum Erhalt von wasserfreier amorpher Trehalose durchgeführt wird.

9. Verfahren nach Anspruch 7 oder 8, worin das Erhitzen bei 50 - 105 °C durchgeführt wird.

10. Verfahren nach Anspruch 9, worin das Erhitzen bei 60 - 90 °C durchgeführt wird.

11. Verfahren nach Anspruch 10, worin das Erhitzen bei 70 °C durchgeführt wird.

12. Verfahren nach einem der vorangehenden Ansprüche, worin die Tablette ferner Trehalosedihydrat umfasst.

## Revendications

1. Méthode de fabrication d'un comprimé, comprenant les étapes qui consistent à :
a) combiner, sous une forme substantiellement sèche, une quantité thérapeutiquement efficace d'un agent actif avec une quantité de tréhalose amorphe anhydre, suffisante pour agir en tant que diluant ;
b) mélanger les composants secs de l'étape a) pour former des poudres, pastilles, granules ou microgranules substantiellement homogènes ; et
c) former un comprimé à partir du produit de l'étape b) dans laquelle le comprimé comprend du tréhalose amorphe anhydre.

2. Méthode selon la revendication 1, dans laquelle l'étape (a) consiste, en outre, à combiner au moins un excipient sélectionné parmi les liants, les lubrifiants, les désintégrants, les colorants et les aromatisants.

3. Méthode selon la revendication 2, dans laquelle l'excipient comprend un liant sélectionné parmi l'amidon, la gélatine, les sucres, les gommes naturelles et synthétiques, la polyvinylpyrrolidone, l'amidon hydroxyéthylique et des mélanges de ces derniers.

4. Méthode selon la revendication 2 ou la revendication 3, dans laquelle l'excipient comprend un lubrifiant sélectionné parmi le talc, le stéarate de calcium, le fumarate de stéaryle sodique, l'acide stéarique, l'huile végétale hydrogénée, le polyéthylèneglycol, le stéarate de magnésium et des mélanges de ces derniers.

5. Méthode selon l'une quelconque des revendications précédentes, dans laquelle l'agent actif est sélectionné parmi les antipyrétiques, les anti-inflammatoires, les analgésiques, les antiarthritiques, les antispasmodiques, les antidépresseurs, les antipsychotiques, les tranquillisants, les anxiolytiques, les antimorphiniques, les antiparkinsoniens, les agonistes cholinergiques, les médicaments chimiothérapeutiques, les immunosuppresseurs, les antiviraux, les antibiotiques, les parasiticides, les anorexigènes, les antiémétiques, les anticholinergiques, les antihistaminiques, les antimigraineux, les vasodilatateurs coronaires, les vasodilatateurs cérébraux, les vasodilatateurs périphériques, les agents hormonaux, les contraceptifs, les antithrombotiques, les diurétiques, les antihypertenseurs, les médicaments cardiovasculaires, les opiacés et les vitamines.

6. Méthode selon l'une quelconque des revendications précédentes, dans laquelle le diluant comprend, en outre, du tréhalose cristallin anhydre.

7. Méthode selon la revendication 6, dans laquelle le tréhalose anhydre est produit en chauffant du dihydrate de tréhalose sous des conditions suffisantes pour que la teneur en eau soit réduite jusqu'à 1 % à 2 %.

8. Méthode selon la revendication 7, dans laquelle le chauffage est réalisé sous une pression de 0,1333 à 3999,671 Pa (1 - 30.000 mTorr) afin de produire du tréhalose amorphe anhydre.

9. Méthode selon la revendication 7 ou la revendication 8, dans laquelle le chauffage est réalisé entre 50 et 105° C.

10. Méthode selon la revendication 9, dans laquelle le chauffage est réalisé entre 60 et 90° C.

11. Méthode selon la revendication 10, dans laquelle le chauffage est réalisé à 70° C.

12. Méthode selon l'une quelconque des revendications précédentes, dans laquelle le comprimé contient, en outre, du dihydrate de tréhalose.
